# EUROPÄISCHE PATENTSCHRIFT

(11) **EP 0 668 917 B1**
(45) Veröffentlichungstag und Bekanntmachung des Hinweises auf die Patenterteilung: **07.05.1997**
(21) Anmeldenummer: 94901793.3
(22) Anmeldetag: 12.11.1993
(51) Int. Cl.: C12N 15/52, C12N 15/53, C12N 15/54, C12N 15/55, C12N 1/19

(54) **RIBOFLAVIN-SYNTHESE IN HEFEN**
RIBOFLAVIN SYNTHESIS IN YEAST
SYNTHESE DE RIBOFLAVINE DANS DES LEVURES

(30) Priorität: 19.11.1992 DE 4238904
(43) Veröffentlichungstag der Anmeldung: 30.08.1995
(73) Patentinhaber: BASF Aktiengesellschaft, 67063 Ludwigshafen (DE)
(72) Erfinder: REVUELTA DOVAL, José Luis, E-37001 Salamanca (ES); SANTOS GARCIA, Maria Angeles, E-37009 Santa Marta (ES); GARCIA-RAMIREZ, José Javier, E-26190 Nalda (ES); GONZALEZ-HERNANDEZ, Gloria Angélica, 200070 Aquascalientes (MX); BUITRAGO SERNA, Maria José, E-37004 Salamanca (ES)
(86) Internationale Anmeldenummer: EP9303183
(87) Internationale Veröffentlichungsnummer: WO9411515

(56) Entgegenhaltungen:
- EP-A- 0 405 370
- EP-A- 0 569 806
- 'From genes to bioproducts' 1990 , DM PPU , MURCIA, SPANIEN BIOTEC 1990, Revuelta, J. L. et al.: Biosynthesis of vitamin B2 in yeast siehe Seite 117 - Seite 122; Abbildungen 2,3
- YEAST (SPEC. ISSUE) Bd. 6 , 1990 Seite S330 SANTOS, M. A. ET AL. 'Molecular characterization of the riboflavin synthase-encoding gene, RIB5, from SACCHAROMYCES CEREVISIAE'
- 'Flavins and Flavoproteins' 1991 , WALTER DE GRUYTER , BERLIN, NEW YORK Proc. Int. Symp., 10th, 1990 Revuelta J. L. et al.: Cloning and molecular characterization of the riboflavin synthase-encoding gene of Saccharomyces cerevisiae siehe Seite 81 - Seite 84
- CURRENT GENETICS Bd. 14, Nr. 5 , November 1988 Seiten 419 - 423 MARIA DE LOS ANGELES SANTOS ET AL. 'Mapping of the RIB5 gene in SACCHAROMYCES CEREVISIAE using UV light as an enhancer of rad52-mediated chromosome loss'
- GENE Bd. 24, Nr. 1 , September 1983 , AMSTERDAM NL Seiten 1 - 14 MELLOR, J. ET AL. 'Efficient synthesis of enzymatically active calf chymosin in SACCAROMYCES CEREVISIAE'
- YEAST Bd. 9, Nr. 10 , Oktober 1993 Seiten 1099 - 1102 BUITRAGO, M.-J. ET AL. 'Mapping of the RIB1 and RIB7 genes involved in the biosynthesis of riboflavin in SACCHAROMYCES CEREVISIAE'
- YEAST Bd. 9, Nr. 2 , Februar 1993 Seiten 189 - 199 DOIGNON, F. ET AL. 'The complete sequence of a 19,482 bp segment located on the right arm of chromosome II from SACCHAROMYCES CEREVISIAE'
- YEAST Bd. 9, Nr. 3 , März 1993 Seiten 289 - 293 BAUR, A. ET AL. 'Sequence of a 4.8 kb fragment of SACCHAROMYCES CEREVISIAE chromosme II including three essential open reading frames'
- JOURNAL OF BACTERIOLOGY Bd. 110, Nr. 3 , 1972 Seiten 818 - 822 OLTMANNS O. UND A. BACHER 'Biosynthesis of riboflavine in SACCHAROMYCES CEREVISIAE: the role of genes rib1 and rib 7'

### SEQUENZ PROTOKOLL

(1) ALLGEMEINE INFORMATION:
   (i) ANMELDER:
      (A) NAME: BASF Aktiengesellschaft
      (B) STRASSE: Carl-Bosch-Strasse 38
      (C) ORT: Ludwigshafen
      (E) LAND: Bundesrepublik Deutschland
      (F) POSTLEITZAHL: D-67056
      (G) TELEPHON: 0621/6048526
      (H) TELEFAX: 0621/6043123
      (I) TELEX: 1762175170
   (ii) ANMELDETITEL: Riboflavin-Synthese in Hefen
   (iii) ANZAHL DER SEQUENZEN: 8
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 1747 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNGLICHE HERKUNFT:
      (A) ORGANISMUS: Saccharomyces cerevisiae
   (ix) MERKMALE:
      (A) NAME/SCHLÜSSEL: 5'UTR
      (B) LAGE: 1..258
   (ix) MERKMALE:
      (A) NAME/SCHLÜSSEL: CDS
      (B) LAGE: 259..1296
   (ix) MERKMALE:
      (A) NAME/SCHLÜSSEL: 3'UTR
      (B) LAGE: 1297..1747
   (xi) SEQUENZBESCHREIBUNG: SEQ ID NO: 1:
   TCTAGACTTA GCATAGGGCT AAAACAGAAA TAGTAGGGTC TATACCGTTT TTGAAAACAA      60
   GTTGTGTAGG GTGAGACTTT TTCTTTTTCC TTTGAGTTTT TCTTTTTCTT TTTACGATTT     120
   GTCAGAAATT AGTCGGACGC TCTTGTGGGG TGAGCGCCAG GCCGCTATAG TATATATAAA     180
   GAAGTTGCTG TGCTGGCGTG GACAATAATT GAGTCATTAC AAAAAGTGGC GTACATAAAA     240
   CTACAACAAA CCTACAGG ATG ACC ATA GAT AAC TAC GAC AAC AGT AAA CAG       291
                       Met Thr Ile Asp Asn Tyr Asp Asn Ser Lys Gln
                        1               5                  10
   GAT AGC AGC AAA TAC GAG GTT AGT GGT ACG GGT GAT GGC AGG AAC GGC     339
   Asp Ser Ser Lys Tyr Glu Val Ser Gly Thr Gly Asp Gly Arg Asn Gly
                15                  20                  25
   GAT GGC GGC TTG CCT CTA GTA CAA TGT GTC GCA AGA GCT CGT ATC CCA     387
   Asp Gly Gly Leu Pro Leu Val Gln Cys Val Ala Arg Ala Arg Ile Pro
            30                  35                  40
   ACC ACA CAG GGT CCG GAT ATC TTT TTA CAT CTT TAC AGT AAC AAC AGG     435
   Thr Thr Gln Gly Pro Asp Ile Phe Leu His Leu Tyr Ser Asn Asn Arg
        45                  50                  55
   GAC AAC AAG GAA CAT CTA GCC ATT GTG TTT GGT GAA GAC ATA CGG TCG     483
   Asp Asn Lys Glu His Leu Ala Ile Val Phe Gly Glu Asp Ile Arg Ser
    60                  65                  70                  75
   CGC TCG CTA TTC CGT AGA AGA CAG TGC GAG ACG CAA CAA GAT AGA ATG     531
   Arg Ser Leu Phe Arg Arg Arg Gln Cys Glu Thr Gln Gln Asp Arg Met
                    80                  85                  90
   ATC AGG GGC GCT TAT ATT GGC AAA CTG TAT CCC GGC AGA ACT GTG GCA     579
   Ile Arg Gly Ala Tyr Ile Gly Lys Leu Tyr Pro Gly Arg Thr Val Ala
                95                 100                 105
   GAC GAA GAC GAT AGA CTC GGA TTA GCT CTG GAG TTT GAT GAT AGT ACA     627
   Asp Glu Asp Asp Arg Leu Gly Leu Ala Leu Glu Phe Asp Asp Ser Thr
           110                 115                 120
   GGT GAG TTA TTA GCT TCC AAA GCC ACT ACA TGG GAC GCC CAT AAC GAC     675
   Gly Glu Leu Leu Ala Ser Lys Ala Thr Thr Trp Asp Ala His Asn Asp
       125                 130                 135
   ACG CTG GTA CGG ATC CAT TCT GAA TGT TAC ACC GGT GAA AAC GCA TGG     723
   Thr Leu Val Arg Ile His Ser Glu Cys Tyr Thr Gly Glu Asn Ala Trp
   140                 145                 150                 155
   AGC GCC CGT TGT GAT TGT GGT GAA CAA TTC GAT AGG GCC GGT AGG CTT     771
   Ser Ala Arg Cys Asp Cys Gly Glu Gln Phe Asp Arg Ala Gly Arg Leu
                   160                 165                 170
   ATC GCT TGC GAC CAC GAA CCC ACA AGC AAC ATC AAA GGT GGA AAC GGC     819
   Ile Ala Cys Asp His Glu Pro Thr Ser Asn Ile Lys Gly Gly Asn Gly
               175                 180                 185
   CAT GGT GTT ATC GTG TAT CTA AGA CAA GAG GGT CGT GGC ATC GGG TTA     867
   His Gly Val Ile Val Tyr Leu Arg Gln Glu Gly Arg Gly Ile Gly Leu
           190                 195                 200
   GGT GAG AAA CTC AAG GCC TAC AAC CTG CAA GAC TTA GGT GCT GAC ACA     915
   Gly Glu Lys Leu Lys Ala Tyr Asn Leu Gln Asp Leu Gly Ala Asp Thr
       205                 210                 215
   GTG CAG GCC AAC TTA ATG CTG AAA CAT CCC GTG GAT GCT AGG GAC TTC     963
   Val Gln Ala Asn Leu Met Leu Lys His Pro Val Asp Ala Arg Asp Phe
   220                 225                 230                 235
   TCG CTG GGT AAG GCT ATT TTG CTG GAT CTT GGC ATC GGT AAT GTC AGG    1011
   Ser Leu Gly Lys Ala Ile Leu Leu Asp Leu Gly Ile Gly Asn Val Arg
                   240                 245                 250
   CTG CTG ACA AAT AAC CCG GAA AAG ATA AAA CAG GTA GAT CAT GCG CCT       1059
   Leu Leu Thr Asn Asn Pro Glu Lys Ile Lys Gln Val Asp His Ala Pro
               255                 260                 265
   TAC CTT AAG TGC GTT GAA CGA GTG CCT ATG GTA CCC ATA CAC TGG ACA       1107
   Tyr Leu Lys Cys Val Glu Arg Val Pro Met Val Pro Ile His Trp Thr
           270                 275                 280
   AAC TCC AGT GAA GGC ATA GAC TCC AAG GAG ATA GAA GGT TAT CTC AGG       1155
   Asn Ser Ser Glu Gly Ile Asp Ser Lys Glu Ile Glu Gly Tyr Leu Arg
       285                 290                 295
   ACC AAG ATA GAA AGA ATG GGT CAT TTA CTA ACG GAG CCT CTG AAA CTT       1203
   Thr Lys Ile Glu Arg Met Gly His Leu Leu Thr Glu Pro Leu Lys Leu
   300                 305                 310                 315
   CAT ACA AAC CCT CAA CCT ACT GAG ACA AGT GAA GCC CAA AAC CAA AAC       1251
   His Thr Asn Pro Gln Pro Thr Glu Thr Ser Glu Ala Gln Asn Gln Asn
                   320                 325                 330
   CGT ATG AAC TCT GCG TTG TCA TCA ACA TCG ACG CTG GCA ATA TAAACACAAG   1303
   Arg Met Asn Ser Ala Leu Ser Ser Thr Ser Thr Leu Ala Ile
               335                 340                 345
   TGTAGCATAA TTATTAGTAC ATATAGTGCT TTTTTTGTAA TGAAACGAAA ATACCATGTT    1363
   ATTTTAATGC GTTACCCCGT CGGCGCGTAT TGGCGCATTA ACATCTTTAT AGAAAATCAA    1423
   ATTATTTACT TATTTATTCT AAAACGCTAA ACATCTATTT ATTTACATTT GAGGTTGAAA    1483
   CTTCCCTTCA TCCGATTGAC AGGCATATTT AGCGGTAATT ATTAGGGTTT TTGGAGAGAC    1543
   CTTGTATTCT TCAGAAATAA TGTCGTCCTT CAACAATGAG ACCAATAATA ACAGCAAACA    1603
   CTAATACACA TCCAGACGAT TCCTTCCCTT TGTATACCGT ATTCAAGGAC GAGTCTGTAC    1663
   CCATCGAGGA AAAAATGGCA CTGCTTACAC GGTTCAAAGG ACATGTAAAA AAGGAACTAG    1723
   TTAACGAATC GTCGATCCAA GCTT                                           1747
(2) INFORMATION ZU SEQ ID NO: 2:
   (i) SEQUENZ CHARAKTERISTIKA:
      (A) LÄNGE: 345 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Thr Ile Asp Asn Tyr Asp Asn Ser Lys Gln Asp Ser Ser Lys Tyr
    1               5                  10                  15
   Glu Val Ser Gly Thr Gly Asp Gly Arg Asn Gly Asp Gly Gly Leu Pro
               20                  25                  30
   Leu Val Gln Cys Val Ala Arg Ala Arg Ile Pro Thr Thr Gln Gly Pro
           35                  40                  45
   Asp Ile Phe Leu His Leu Tyr Ser Asn Asn Arg Asp Asn Lys Glu His
       50                  55                  60
   Leu Ala Ile Val Phe Gly Glu Asp Ile Arg Ser Arg Ser Leu Phe Arg
   65                  70                  75                  80
   Arg Arg Gln Cys Glu Thr Gln Gln Asp Arg Met Ile Arg Gly Ala Tyr
                   85                  90                  95
   Ile Gly Lys Leu Tyr Pro Gly Arg Thr Val Ala Asp Glu Asp Asp Arg
               100                 105                 110
   Leu Gly Leu Ala Leu Glu Phe Asp Asp Ser Thr Gly Glu Leu Leu Ala
           115                 120                 125
   Ser Lys Ala Thr Thr Trp Asp Ala His Asn Asp Thr Leu Val Arg Ile
       130                 135                 140
   His Ser Glu Cys Tyr Thr Gly Glu Asn Ala Trp Ser Ala Arg Cys Asp
   145                 150                 155                 160
   Cys Gly Glu Gln Phe Asp Arg Ala Gly Arg Leu Ile Ala Cys Asp His
                   165                 170                 175
   Glu Pro Thr Ser Asn Ile Lys Gly Gly Asn Gly His Gly Val Ile Val
               180                 185                 190
   Tyr Leu Arg Gln Glu Gly Arg Gly Ile Gly Leu Gly Glu Lys Leu Lys
           195                 200                 205
   Ala Tyr Asn Leu Gln Asp Leu Gly Ala Asp Thr Val Gln Ala Asn Leu
       210                 215                 220
   Met Leu Lys His Pro Val Asp Ala Arg Asp Phe Ser Leu Gly Lys Ala
   225                 230                 235                 240
   Ile Leu Leu Asp Leu Gly Ile Gly Asn Val Arg Leu Leu Thr Asn Asn
                   245                 250                 255
   Pro Glu Lys Ile Lys Gln Val Asp His Ala Pro Tyr Leu Lys Cys Val
               260                 265                 270
   Glu Arg Val Pro Met Val Pro Ile His Trp Thr Asn Ser Ser Glu Gly
           275                 280                 285
   Ile Asp Ser Lys Glu Ile Glu Gly Tyr Leu Arg Thr Lys Ile Glu Arg
       290                 295                 300
   Met Gly His Leu Leu Thr Glu Pro Leu Lys Leu His Thr Asn Pro Gln
   305                 310                 315                 320
   Pro Thr Glu Thr Ser Glu Ala Gln Asn Gln Asn Arg Met Asn Ser Ala
                   325                 330                 335
   Leu Ser Ser Thr Ser Thr Leu Ala Ile
               340                 345
(2) INFORMATION ZU SEQ ID NO: 3:
   (i) SEQUENZ CHARAKTERISTIKA:
      (A) LÄNGE: 3086 Basenpaare
      (B) ART: Nukleinsaure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNGLICHE HERKUNFT:
      (A) ORGANISMUS: Saccharomyces cerevisiae
   (ix) MERKMALE:
      (A) NAME/SCHLÜSSEL: 5'UTR
      (B) LAGE: 1..592
   (ix) MERKMALE:
      (A) NAME/SCHLÜSSEL: CDS
      (B) LAGE: 593..2368
   (ix) MERKMALE:
      (A) NAME/SCHLÜSSEL: 3'UTR
      (B) LAGE: 2369..3086
   (xi) SEQUENZBESCHREIBUNG: SEQ ID NO: 3:
   ATGAGCCCGA AGTGGGAGCG CGCGATCTTG CCCCATCGGT GATGTCGGCG ATATAGGCGC      60
   CAGACACCGC ACCTGTGGCG CCGGTGATGC CGCACGATGC GTCCGGCGTA GAGGATCATC     120
   AACCCGTTAA TCTTACCGTA AGATTACCAA GGTCTACAGG GACGCCAGTG CCTTATCCTT     180
   ACATATCGAA AGTATCCTTT ATCATGGAGC TCTGGCCTGA TTGGACAAAT TGGTGACGCA     240
   GTCATCGGGT ACTGCGGTTG GCTAGTTACT AAGAATGTAC ATTATTGGAT ATTAGGTTCT     300
   TTACTATTAT ATTTACTATT AAAAATCCTG TAAAGTGATC TGGGCTGTCA GGCTAGAAGT     360
   TTTTCATTTA TGTCTGAAAC AGATAACGTC AGTTGTACCA CTATCTTTGG TTTTATTCTT     420
   TTTATTTTCT CTTCCATATA TCTTTACAGA ACTTAAGTAA TAATGAAAAA GAATGAAAAA     480
   AAAAAAAAAA AAAGAAAAAC TAGTCAAAAT TATTGCCATG AAAAAACAGG AAGAAGAGAG     540
   AAGAACATTA AAAAAAAAAA ACTATCAATA TTAGATTAAC AAACCAATCA AA ATG         595
                                                             Met
                                                               1
   GAG GAC TCT AAT AAT GAA GCC AGC GAT GAC TTC AAC AAC TTA TTA AAC       643
   Glu Asp Ser Asn Asn Glu Ala Ser Asp Asp Phe Asn Asn Leu Leu Asn
               5                  10                  15
   AAA GAA ATT GAG TCT GCA AAG GAA GTG AAG TTA AGA AAA TTC GCG AAC       691
   Lys Glu Ile Glu Ser Ala Lys Glu Val Lys Leu Arg Lys Phe Ala Asn
           20                  25                  30
   AGG AAT AAT AAT AGG AAC GAA AAC AGT TCC AAA GTC AAA GAT GCG AGT       739
   Arg Asn Asn Asn Arg Asn Glu Asn Ser Ser Lys Val Lys Asp Ala Ser
       35                  40                  45
   GGA TTC AGA CTG AGA GTT ATT CAA ACC GAT GGG CAC AAA ACT AAA AAG       787
   Gly Phe Arg Leu Arg Val Ile Gln Thr Asp Gly His Lys Thr Lys Lys
    50                  55                  60                  65
   ACA GAC CCT GAT TAT GAA GTG ACA ATT GAT GGA CCC TTA AGG AAG ATT       835
   Thr Asp Pro Asp Tyr Glu Val Thr Ile Asp Gly Pro Leu Arg Lys Ile
                    70                  75                  80
   GAG CCT TAT TTT TTC ACT TAT AAA ACT TTC TGT AAG GAG AGG TGG AGA       883
   Glu Pro Tyr Phe Phe Thr Tyr Lys Thr Phe Cys Lys Glu Arg Trp Arg
                85                  90                  95
   GAT CGT AAG TTG GTG GAT GTA TTC GTG AGC GAA TTC AGG GAT CGA GAG       931
   Asp Arg Lys Leu Val Asp Val Phe Val Ser Glu Phe Arg Asp Arg Glu
           100                 105                 110
   CCC AGC TAT TAT TCC AAA ACC ATT GCA GAG GGA AAA GTA TAC TTA AAC       979
   Pro Ser Tyr Tyr Ser Lys Thr Ile Ala Glu Gly Lys Val Tyr Leu Asn
       115                 120                 125
   GAT GAA CCT GCA AAC CTT GAT ACC ATC ATT CGT GAC GGT GAT CTG ATC      1027
   Asp Glu Pro Ala Asn Leu Asp Thr Ile Ile Arg Asp Gly Asp Leu Ile
   130                 135                 140                 145
   ACA CAT AAA GTA CAT CGA CAT GAA CCG CCA GTC ACA TCT AAA CCA ATA      1075
   Thr His Lys Val His Arg His Glu Pro Pro Val Thr Ser Lys Pro Ile
                   150                 155                 160
   GAT ATT GTG TTT GAA GAC GAA GAT ATC CTT GTC ATT GAT AAA CCC AGC     1123
   Asp Ile Val Phe Glu Asp Glu Asp Ile Leu Val Ile Asp Lys Pro Ser
               165                 170                 175
   AGT ATT CCA GTG CAC CCA ACG GGC AGA TAC AGA TTC AAT ACC ATT ACA     1171
   Ser Ile Pro Val His Pro Thr Gly Arg Tyr Arg Phe Asn Thr Ile Thr
           180                 185                 190
   AAA ATG CTA GAA AGA CAG CTA GGT TAT TCA GTA CAT CCA TGT AAT AGA     1219
   Lys Met Leu Glu Arg Gln Leu Gly Tyr Ser Val His Pro Cys Asn Arg
       195                 200                 205
   CTA GAC AAG CCA ACT AGT GGA CTA ATG TTT TTA GCC AAG ACT CCA TTA     1267
   Leu Asp Lys Pro Thr Ser Gly Leu Met Phe Leu Ala Lys Thr Pro Leu
   210                 215                 220                 225
   GGT GCA GAT AGA ATG GGA GAC CAA ATG AAA GCG AGG GAA GTC ACC AAA     1315
   Gly Ala Asp Arg Met Gly Asp Gln Met Lys Ala Arg Glu Val Thr Lys
                   230                 235                 240
   GAA TAC GTA GCC CGC GTG AAG GGC GAG TTC CCT ATA GGT ATA GTA GAG     1363
   Glu Tyr Val Ala Arg Val Lys Gly Glu Phe Pro Ile Gly Ile Val Glu
               245                 250                 255
   GTA GAT AAG CCT GTC AGA TCT GTT AAC CCT AAA GTT GCA CTA AAC GCT     1411
   Val Asp Lys Pro Val Arg Ser Val Asn Pro Lys Val Ala Leu Asn Ala
           260                 265                 270
   GTT TGC GAG ATG AGC GAT GAA AAC GCT AAA CAT GCC AAG ACC GTT TTC     1459
   Val Cys Glu Met Ser Asp Glu Asn Ala Lys His Ala Lys Thr Val Phe
       275                 280                 285
   CAA AGG GTT AGC TAT GAT GGA CAG ACG AGT ATT GTA AAG TGT AAA CCA     1507
   Gln Arg Val Ser Tyr Asp Gly Gln Thr Ser Ile Val Lys Cys Lys Pro
   290                 295                 300                 305
   TTG ACA GGT AGA ACT CAC CAA ATA AGA GTC CAT TTG CAG TAC TTG GGA     1555
   Leu Thr Gly Arg Thr His Gln Ile Arg Val His Leu Gln Tyr Leu Gly
                   310                 315                 320
   TTT CCC ATA GCA AAT GAC CCT ATA TAC TCG AAC CCA GAT ATC TGG GGT     1603
   Phe Pro Ile Ala Asn Asp Pro Ile Tyr Ser Asn Pro Asp Ile Trp Gly
               325                 330                 335
   CCA GAT TTG GGC CGC GGT GGA CTT CAA AAC TAT GAT GAC ATC GTT CTG     1651
   Pro Asp Leu Gly Arg Gly Gly Leu Gln Asn Tyr Asp Asp Ile Val Leu
           340                 345                 350
   AAA CTA GAC GCT ATT GGC AAG ACT AAT CCT GCA GAG AGC TGG ATT CAT     1699
   Lys Leu Asp Ala Ile Gly Lys Thr Asn Pro Ala Glu Ser Trp Ile His
       355                 360                 365
   CCT CAT AGC GAG GGT GAA TAC TTG CTT GGC CGT CAG TGC GAA GAA TGC     1747
   Pro His Ser Glu Gly Glu Tyr Leu Leu Gly Arg Gln Cys Glu Glu Cys
   370                 375                 380                 385
   GAG GCT GAA ATG TAC ACA GAT CCC GGT ACT AAT GAT CTC GAC CTC TGG     1795
   Glu Ala Glu Met Tyr Thr Asp Pro Gly Thr Asn Asp Leu Asp Leu Trp
                   390                 395                 400
   CTG CAT GCC TTC CGG TAC GAG TCA TTG GAA AGA AAT TCG GAT ACG CAA     1843
   Leu His Ala Phe Arg Tyr Glu Ser Leu Glu Arg Asn Ser Asp Thr Gln
                   405                 410                 415
   AAA CCT CTC TGG AGT TAT AGA ACA AAA TAC CCT GAA TGG GCC TTA GAG     1891
   Lys Pro Leu Trp Ser Tyr Arg Thr Lys Tyr Pro Glu Trp Ala Leu Glu
               420                 425                 430
   CCA CAT CGC CGA TAT ATG GAA ATG GCC GTT AAG GAA GCT GGT AAG TGT     1939
   Pro His Arg Arg Tyr Met Glu Met Ala Val Lys Glu Ala Gly Lys Cys
       435                 440                 445
   GGC CCG ACA AAG ACT GCT TTT AGT GTT GGT GCC GTT CTT GTT CAT GGA     1987
   Gly Pro Thr Lys Thr Ala Phe Ser Val Gly Ala Val Leu Val His Gly
   450                 455                 460                 465
   ACT CAA GTA CTT GCC ACA GGA TAT TCA AGA GAG CTA CCA GGA AAC ACT     2035
   Thr Gln Val Leu Ala Thr Gly Tyr Ser Arg Glu Leu Pro Gly Asn Thr
                   470                 475                 480
   CAT GCA GAA CAG TGT GCT TTG ATA AAG TAC TCG CAG TTA CAC CCG AAC     2083
   His Ala Glu Gln Cys Ala Leu Ile Lys Tyr Ser Gln Leu His Pro Asn
               485                 490                 495
   TGT CCC ACT ATA GTC CCT ATG GGA ACA GTG CTC TAT ACA ACC ATG GAA     2131
   Cys Pro Thr Ile Val Pro Met Gly Thr Val Leu Tyr Thr Thr Met Glu
           500                 505                 510
   CCC TGT TCC TTC AGG CTA AGT GGC AAT GAG CCT TGC TGC GAT AGA ATC     2179
   Pro Cys Ser Phe Arg Leu Ser Gly Asn Glu Pro Cys Cys Asp Arg Ile
       515                 520                 525
   CTG GCC ACC CAA GGT GCC ATT GGT ACT GTC TTC GTG GGG GTT ATG GAG     2227
   Leu Ala Thr Gln Gly Ala Ile Gly Thr Val Phe Val Gly Val Met Glu
   530                 535                 540                 545
   CCC GAT ACA TTT GTT AAA AAC AAT ACA AGT TTG AAC AAG CTG GAA TCG     2275
   Pro Asp Thr Phe Val Lys Asn Asn Thr Ser Leu Asn Lys Leu Glu Ser
                   550                 555                 560
   CAC GGT GTG AAC TAC ATA CAA ATA CCA GGC TAC GAG GAG GAG TGC ACC     2323
   His Gly Val Asn Tyr Ile Gln Ile Pro Gly Tyr Glu Glu Glu Cys Thr
               565                 570                 575
   ATC ATT GCC TTC AAA GGC CAC GAT AAT AGT GAC GAC AAA GCT TAGACTATTT  2375
   Ile Ile Ala Phe Lys Gly His Asp Asn Ser Asp Asp Lys Ala
           580                 585                 590
   AGCATATATA TTATCATGTA ATCTTACATC ATATCTACAA TAACCACTCA TTACTGGAAA   2435
   CGGAAAAAGG CGCCACCGAA AATTTTGAGT AAAAAGAATG GAATTAACTT CAACCACAAC   2495
   ACAGACAAAA AACACTAGAT AGTGAACCAA AAGAAAGCAC AACACCAAAC ATGAGCAGCA   2555
   TTCCAGCTGG CACTGATCCT GGGTCCTGCG GTGCTAATTT CAAGAATGAC CGCAAGCGCA   2615
   GAGATAAGAT CAACGACCGT ATTCAAGAAC TATTGAGTAT CATTCCCAAA GACTTCTTTA   2675
   GAGATTATTA CGGCAATTCT GGTAGCAATG ACACGTTAAG TGAATCCACT CCCGGTGCGC   2735
   TTGGGTTGTC CAGCAAGGCC AAAGGTACAG GGACCAAGGA CGGAAAGCCC AACAAGGGCC   2795
   AAATTCTCAC ACAGGCGGTA GAGTACATAT CACATCTACA AAATCAAGTG GACACACAGA   2855
   ACAGAGAGGA GGTGGAACTG ATGGTGAAGG CCACTCAGTT GGCCAAGCAG ACAGGCACCA   2915
   TTGTCAACGA TATAAACTTA GAGAACACCA GCGCTGAAGT CGCCCTGTCC AGGATTGGCG   2975
   TGGGACCGCT GGCCGCAACA AATGATGACT CAGTAAGACC GCCAGCAAAG AGGTTGAGCT   3035
   CCTTCGAGTA CGGAGGGTAT GGTGAGTACG GTAATGGTAG CTAAAAGTAC T 3086
(2) INFORMATION ZU SEQ ID NO: 4:
   (i) SEQUENZ CHARAKTERISTIKA:
      (A) LÄNGE: 591 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Glu Asp Ser Asn Asn Glu Ala Ser Asp Asp Phe Asn Asn Leu Leu
    1               5                  10                  15
   Asn Lys Glu Ile Glu Ser Ala Lys Glu Val Lys Leu Arg Lys Phe Ala
               20                  25                  30
   Asn Arg Asn Asn Asn Arg Asn Glu Asn Ser Ser Lys Val Lys Asp Ala
           35                  40                  45
   Ser Gly Phe Arg Leu Arg Val Ile Gln Thr Asp Gly His Lys Thr Lys
       50                  55                  60
   Lys Thr Asp Pro Asp Tyr Glu Val Thr Ile Asp Gly Pro Leu Arg Lys
   65                  70                  75                  80
   Ile Glu Pro Tyr Phe Phe Thr Tyr Lys Thr Phe Cys Lys Glu Arg Trp
                   85                  90                  95
   Arg Asp Arg Lys Leu Val Asp Val Phe Val Ser Glu Phe Arg Asp Arg
               100                 105                 110
   Glu Pro Ser Tyr Tyr Ser Lys Thr Ile Ala Glu Gly Lys Val Tyr Leu
           115                 120                 125
   Asn Asp Glu Pro Ala Asn Leu Asp Thr Ile Ile Arg Asp Gly Asp Leu
       130                 135                 140
   Ile Thr His Lys Val His Arg His Glu Pro Pro Val Thr Ser Lys Pro
   145                 150                 155                 160
   Ile Asp Ile Val Phe Glu Asp Glu Asp Ile Leu Val Ile Asp Lys Pro
                   165                 170                 175
   Ser Ser Ile Pro Val His Pro Thr Gly Arg Tyr Arg Phe Asn Thr Ile
               180                 185                 190
   Thr Lys Met Leu Glu Arg Gln Leu Gly Tyr Ser Val His Pro Cys Asn
           195                 200                 205
   Arg Leu Asp Lys Pro Thr Ser Gly Leu Met Phe Leu Ala Lys Thr Pro
       210                 215                 220
   Leu Gly Ala Asp Arg Met Gly Asp Gln Met Lys Ala Arg Glu Val Thr
   225                 230                 235                 240
   Lys Glu Tyr Val Ala Arg Val Lys Gly Glu Phe Pro Ile Gly Ile Val
                   245                 250                 255
   Glu Val Asp Lys Pro Val Arg Ser Val Asn Pro Lys Val Ala Leu Asn
               260                 265                 270
   Ala Val Cys Glu Met Ser Asp Glu Asn Ala Lys His Ala Lys Thr Val
           275                 280                 285
   Phe Gln Arg Val Ser Tyr Asp Gly Gln Thr Ser Ile Val Lys Cys Lys
       290                 295                 300
   Pro Leu Thr Gly Arg Thr His Gln Ile Arg Val His Leu Gln Tyr Leu
   305                 310                 315                 320
   Gly Phe Pro Ile Ala Asn Asp Pro Ile Tyr Ser Asn Pro Asp Ile Trp
                   325                 330                 335
   Gly Pro Asp Leu Gly Arg Gly Gly Leu Gln Asn Tyr Asp Asp Ile Val
               340                 345                 350
   Leu Lys Leu Asp Ala Ile Gly Lys Thr Asn Pro Ala Glu Ser Trp Ile
           355                 360                 365
   His Pro His Ser Glu Gly Glu Tyr Leu Leu Gly Arg Gln Cys Glu Glu
       370                 375                 380
   Cys Glu Ala Glu Met Tyr Thr Asp Pro Gly Thr Asn Asp Leu Asp Leu
   385                 390                 395                 400
   Trp Leu His Ala Phe Arg Tyr Glu Ser Leu Glu Arg Asn Ser Asp Thr
                   405                 410                 415
   Gln Lys Pro Leu Trp Ser Tyr Arg Thr Lys Tyr Pro Glu Trp Ala Leu
               420                 425                 430
   Glu Pro His Arg Arg Tyr Met Glu Met Ala Val Lys Glu Ala Gly Lys
           435                 440                 445
   Cys Gly Pro Thr Lys Thr Ala Phe Ser Val Gly Ala Val Leu Val His
       450                 455                 460
   Gly Thr Gln Val Leu Ala Thr Gly Tyr Ser Arg Glu Leu Pro Gly Asn
   465                 470                 475                 480
   Thr His Ala Glu Gln Cys Ala Leu Ile Lys Tyr Ser Gln Leu His Pro
                   485                 490                 495
   Asn Cys Pro Thr Ile Val Pro Met Gly Thr Val Leu Tyr Thr Thr Met
               500                 505                 510
   Glu Pro Cys Ser Phe Arg Leu Ser Gly Asn Glu Pro Cys Cys Asp Arg
           515                 520                 525
   Ile Leu Ala Thr Gln Gly Ala Ile Gly Thr Val Phe Val Gly Val Met
       530                 535                 540
   Glu Pro Asp Thr Phe Val Lys Asn Asn Thr Ser Leu Asn Lys Leu Glu
   545                 550                 555                 560
   Ser His Gly Val Asn Tyr Ile Gln Ile Pro Gly Tyr Glu Glu Glu Cys
                   565                 570                 575
   Thr Ile Ile Ala Phe Lys Gly His Asp Asn Ser Asp Asp Lys Ala
               580                 585                 590
(2) INFORMATION ZU SEQ ID NO: 7:
   (i) SEQUENZ CHARAKTERISTIKA:
      (A) LÄNGE: 1300 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNGLICHE HERKUNFT:
      (A) ORGANISMUS: Saccharomyces cerevisiae
   (ix) MERKMALE:
      (A) NAME/SCHLÜSSEL: 5'UTR
      (B) LAGE: 1..469
   (ix) MERKMALE:
      (A) NAME/SCHLÜSSEL: CDS
      (B) LAGE: 470..979
   (ix) MERKMALE:
      (A) NAME/SCHLÜSSEL: 3'UTR
      (B) LAGE: 980..1300
   (xi) SEQUENZBESCHREIBUNG: SEQ ID NO: 7:
   AATATGAAGC TGGACATCTT GTATGCGCTT GGTTAGTCGA AATGCTGGAG ATGAGCTGCT 60
   GAAGCATCTG TTTGTGCTGT TGGTTTGATA TGCTTCTCTA AACCAAAAAA TTTCAGCCGA 120
   TCAGGTCCGG GTCACACCAC TACCCTTACT AGTAAACAAT GATGGCTTTC ATTAAGGTAA 180
   CTTTAAGTGA GGTGGATGGA AGTAGGTTCC ACGAAACTTC ATCGAAGAGG TCTGGGGTTC 240
   TATGGCATAT AGTTAAAACT CTGAAAGGTT TTCGGAGGTT CCCGTAGTCA TTTCTTAAGG 300
   CTCTATGACA TATTCTGGGA GCGGAGATAT TTTCGGAGGT CCCCGTTAAT AAGAGAAAAG 360
   TAGCCATATA AAAGAGAACA ATGAAATATT GTCCCAGAGA GTGGGTTCAA AAGCATGAAA 420
   AGGAACAGTA TAACGCAGTA TAACGCAGTA TAACGCAGTA TAACGCAGT ATG GCA 475
   Met Ala
   1
   GTT AAA GGA TTA GGC AAA CCA GAT CAA GTT TAT GAC GGT TCC AAA ATC 523
   Val Lys Gly Leu Gly Lys Pro Asp Gln Val Tyr Asp Gly Ser Lys Ile
   5 10 15
   AGG GTC GGT ATC ATT CAT GCC CGT TGG AAC CGT GTC ATT ATT GAC GCT 571
   Arg Val Gly Ile Ile His Ala Arg Trp Asn Arg Val Ile Ile Asp Ala
   20 25 30
   CTT GTA AAA GGT GCT ATT GAA AGA ATG GTT TCC CTT GGT GTC GAG GAA 619
   Leu Val Lys Gly Ala Ile Glu Arg Met Val Ser Leu Gly Val Glu Glu
   35 40 45 50
   AAG AAC ATA ATA ATT GAA ACT GTT CCT GGA TCC TAT GAA TTA CCT TGG 667
   Lys Asn Ile Ile Ile Glu Thr Val Pro Gly Ser Tyr Glu Leu Pro Trp
   55 60 65
   GGG ACA AAA AGG TTT GTT GAC AGA CAA GCG AAG TTG GGA AAG CCG CTA 715
   Gly Thr Lys Arg Phe Val Asp Arg Gln Ala Lys Leu Gly Lys Pro Leu
   70 75 80
   GAT GTT GTA ATT CCT ATT GGT GTA CTT ATC AAA GGT AGT ACA ATG CAC 763
   Asp Val Val Ile Pro Ile Gly Val Leu Ile Lys Gly Ser Thr Met His
   85 90 95
   TTT GAA TAC ATT TCA GAT TCC ACT ACT CAC GCA TTG ATG AAC TTA CAA 811
   Phe Glu Tyr Ile Ser Asp Ser Thr Thr His Ala Leu Met Asn Leu Gln
   100 105 110
   GAA AAG GTC GAC ATG CCC GTC ATT TTT GGC CTC TTA ACT TGC ATG ACT 859
   Glu Lys Val Asp Met Pro Val Ile Phe Gly Leu Leu Thr Cys Met Thr
   115 120 125 130
   GAA GAA CAA GCC CTG GCC AGA GCT GGC ATC GAT GAA GCG CAC TCC ATG 907
   Glu Glu Gln Ala Leu Ala Arg Ala Gly Ile Asp Glu Ala His Ser Met
   135 140 145
   CAC AAC CAT GGT GAA GAT TGG GGT GCT GCA GCA GTG GAA ATG GCT GTT        955
   His Asn His Gly Glu Asp Trp Gly Ala Ala Ala Val Glu Met Ala Val
               150                 155                 160
   AAG TTC GGT AAA AAT GCT TTT TGAATAAGCG CAGAGAAATC TATACTATAT          1006
   Lys Phe Gly Lys Asn Ala Phe
           165                 170
   ATCTATATAT ACTCACTATA GAAGAAGCCC GCTCTTTGAT TGTTTCCTCT TACGGTGCTT    1066
   CAATGCATCT TTCATTTTGC GCCTCATATC ACGGGTCCAA TTACCTCTGT TAGCCCAGAA    1126
   CTCCTCATCC CAGTTCGATA GCATTGTTTC TCTTCCAGGA GCCCTTACTT TACTCAACTG    1186
   TGTTTGTCCT ACTAAAAATG GCGATTGCAA ATGAGGGAAA AACAAGAAGT TTTTGTTATC    1246
   TTTATCCCTG GATACATGTG GTACGGGAGC CGTATGCAAA CCTAGGGTGT CAGA          1300
(2) INFORMATION ZU SEQ ID NO: 8:
   (i) SEQUENZ CHARAKTERISTIKA:
      (A) LÄNGE: 169 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 8:
   Met Ala Val Lys Gly Leu Gly Lys Pro Asp Gln Val Tyr Asp Gly Ser
     1               5                  10                  15
   Lys Ile Arg Val Gly Ile Ile His Ala Arg Trp Asn Arg Val Ile Ile
                20                  25                  30
   Asp Ala Leu Val Lys Gly Ala Ile Glu Arg Met Val Ser Leu Gly Val
            35                  40                  45
   Glu Glu Lys Asn Ile Ile Ile Glu Thr Val Pro Gly Ser Tyr Glu Leu
        50                  55                  60
   Pro Trp Gly Thr Lys Arg Phe Val Asp Arg Gln Ala Lys Leu Gly Lys
    65                  70                  75                  80
   Pro Leu Asp Val Val Ile Pro Ile Gly Val Leu Ile Lys Gly Ser Thr
                    85                  90                  95
   Met His Phe Glu Tyr Ile Ser Asp Ser Thr Thr His Ala Leu Met Asn
               100                 105                 110
   Leu Gln Glu Lys Val Asp Met Pro Val Ile Phe Gly Leu Leu Thr Cys
           115                 120                 125
   Met Thr Glu Glu Gln Ala Leu Ala Arg Ala Gly Ile Asp Glu Ala His
       130                 135                 140
   Ser Met His Asn His Gly Glu Asp Trp Gly Ala Ala Ala Val Glu Met
   145                 150                 155                 160
   Ala Val Lys Phe Gly Lys Asn Ala Phe
                   165
(2) INFORMATION ZU SEQ ID NO: 11:
   (i) SEQUENZ CHARAKTERISTIKA:
      (A) LÄNGE: 2365 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNGLICHE HERKUNFT:
      (A) ORGANISMUS: Saccharomyces cerevisiae
   (ix) MERKMALE:
      (A) NAME/SCHLÜSSEL: 5'UTR
      (B) LAGE: 1..1344
   (ix) MERKMALE:
      (A) NAME/SCHLÜSSEL: CDS
      (B) LAGE: 1345..2079
   (ix) MERKMALE:
      (A) NAME/SCHLÜSSEL: 3'UTR
      (B) LAGE: 2080..2365
   (xi) SEQUENZBESCHREIBUNG: SEQ ID NO: 11:
   TCCTGTATTT CAACTTTCCA TACATGAAAG ATTCTTTGGC CATTTTCTTA ATCCATTTCA     60
   TATTTTCATC TGATTTTTTT TCTGTTTTCC ACTTGCTGCT CATTATTTCA TTTTATAATT    120
   TTTGCAAACA TGCAATATTT CGCCATATAT TGAGAAAAGC AATTCTTCGT TGTTCCCAGT    180
   AAAGAAAGAA CAAATGCAGA TTTCTACCGC CACCGCTAGA TTAAGAATTT TTTTTGGTTA    240
   CTGATTTGAA TCACATACGA TGAGTTTTAG ACATTTCAAG AGGAGACTTG ACACAAGCTC    300
   AGCAGATGAA AGCTCTTCGG CGGATGAAGA GCATCCAGAC CAAAACGTAT CTCTAACAGA    360
   AAAATCAGCC TCATTAAGCC ATAGTGATTT GGGCGGCGAA ATTTTAAATG GTACAGGAAA    420
   GAACCGCACC CCCAATGATG GCCAAGAATC AAATGAAAGT GATGGGAGTC CCGAAAGTGA    480
   TGAGAGTCCC GAAAGTGAAG AAAGTAGCGA CAACAGTGAT TCGAGCGATA GTGACGATAT    540
   GAGACCTTTA CCGAGGCCAT TATTTATGAA GAAAAAGGCC AATAATTTGC AGAAAGCTAC    600
   CAAGATAGAT CAACCCTGGA ATGCCCAAGA TGACGCACGA GTTCTGCAAA CAAAGAAGGA    660
   AAATATGATA AAAAACATCG ACAAAGCTAA TCAAGTGGCA AAGAACTACG AAACGATGAA    720
   GCTGAGACTT AACACCAATT ACAGCACCAA CGAAGAATTG ATCAAGCAGT GCTTGCTCTT    780
   AGATGATAAC GATGAAGTCG ATTCAGAAAA GGAGAGACAG AAATGGTTTG AAAGGCAGAA    840
   CGAACGAAAG CAAAAGCATA GAAGAATACA ACTTGCGAAA CAAAGAGAAT CAGAAGAATA    900
   TGAGGCAAAA CGATTTGAAG CGATGCAAAA AGGCAAAGAT GGGAATACCA AATACGACGT    960
   TATCTTGGAC AAGGAGAAAG AGAAACTTGA TCACAAAAAA CAAAGATCGG CTGAAAAAGT   1020
   AGAAAAATCT CACAATAACA ACCGTTACAA GATTACAAGA ACTAAAAATG TTGAATTTGG  1080
   TGACCTAGGC AAGAATAGTA GAGATTACGA AGAAACTGAA TATTCGGTTA TATAACACCG   1140
   CATCACACAC ACAAACCCAC ATGCCTTTCA AGTTAGTTGA TGTCGGGAAA ATCCCGAAAC   1200
   AGAAAAAAGA GGCAAAGATG AAGATATCAA ATTTTTTCGC AGAACCATTA CATCTGGCTT   1260
   TGGATACGAT GAAACAATCT AGCAGAAATA CAGTACCAAC CTATTGGTAG CTTCTTTGTA   1320
   TTACTATCAA CATTTTTAGA AGAT ATG TCT TTG ACA CCA CTG TGT GAA GAT      1371
                              Met Ser Leu Thr Pro Leu Cys Glu Asp
                               1               5
   TTA CCA CAA TTT CTG CAA AAC TAT CTA CCG AAT GCT GGT CAA ACG GAA     1419
   Leu Pro Gln Phe Leu Gln Asn Tyr Leu Pro Asn Ala Gly Gln Thr Glu
    10                  15                  20                  25
   AAT ACC ATT GTG CCC TTT GTC ACA CTA ACT TAT GCT CAA TCG CTC GAC     1467
   Asn Thr Ile Val Pro Phe Val Thr Leu Thr Tyr Ala Gln Ser Leu Asp
                    30                  35                  40
   GCG AGA GTA TCT AGG GGC CCT GGA GTG AGG ACT ACA ATT TCA CAT CCC      1515
   Ala Arg Val Ser Arg Gly Pro Gly Val Arg Thr Thr Ile Ser His Pro
               45                  50                  55
   GAG ACC AAA ACA ATG ACG CAT TAT TTG AGA CAT CAT CAC GAT GGA ATA      1563
   Glu Thr Lys Thr Met Thr His Tyr Leu Arg His His His Asp Gly Ile
           60                  65                  70
   CTC GTA GGA AGT GGA ACA GTG CTA GCT GAT AAT CCT GGA TTG AAT TGT      1611
   Leu Val Gly Ser Gly Thr Val Leu Ala Asp Asn Pro Gly Leu Asn Cys
       75                  80                  85
   AAA TGG GGT CCC GAT CCG GCT GCA AAT TCC CCA AGG CCA ATA ATA ATA      1659
   Lys Trp Gly Pro Asp Pro Ala Ala Asn Ser Pro Arg Pro Ile Ile Ile
    90                  95                 100                 105
   GAT ACA AAG CAA AAG TGG CGA TTT GAT GGT TCA AAA ATG CAA GAA CTT      1707
   Asp Thr Lys Gln Lys Trp Arg Phe Asp Gly Ser Lys Met Gln Glu Leu
                   110                 115                 120
   TTT ATT AAA CGA CAG GGT AAG CCG CCA ATC GTT GTT GTC ACA AGT GAG      1755
   Phe Ile Lys Arg Gln Gly Lys Pro Pro Ile Val Val Val Thr Ser Glu
               125                 130                 135
   CCC ATT ATA AAA GAA CAA CAT GTA GAC TAC GCA ATT TGT CCA ATA AAT      1803
   Pro Ile Ile Lys Glu Gln His Val Asp Tyr Ala Ile Cys Pro Ile Asn
           140                 145                 150
   GAT ACT ACG AAA TTG GTC GAT TGG AAG AAA TTG TTT GAG ATA TTA AAA      1851
   Asp Thr Thr Lys Leu Val Asp Trp Lys Lys Leu Phe Glu Ile Leu Lys
       155                 160                 165
   GAA GAA TTC AAT ATA AGG TCA GTA ATG GTT GAA GGA GGT GCC AAT GTA      1899
   Glu Glu Phe Asn Ile Arg Ser Val Met Val Glu Gly Gly Ala Asn Val
   170                 175                 180                 185
   ATA AAT CAG TTG TTG CTG AGG AGC GAT ATT GTC AAC AGT CTT ATA ATA      1947
   Ile Asn Gln Leu Leu Leu Arg Ser Asp Ile Val Asn Ser Leu Ile Ile
                   190                 195                 200
   ACT ATT GGA TCA ACA TTT CTG GGT AGC TCA GGC ACC GAA GTT AGC CCA      1995
   Thr Ile Gly Ser Thr Phe Leu Gly Ser Ser Gly Thr Glu Val Ser Pro
               205                 210                 215
   CCC CAA ACA GTA AAT TTA AAG GAT ATG TCA TGG TGG AAG GGC ATT ACC      2043
   Pro Gln Thr Val Asn Leu Lys Asp Met Ser Trp Trp Lys Gly Ile Thr
           220                 225                 230
   GAT GTG GTG CTT TGT GCG AGA CTG GCC GAT GAC TAATAGTGTT AGAACTCCAC    2096
   Asp Val Val Leu Cys Ala Arg Leu Ala Asp Asp
       235                 240                 245
   CTTCTGTGTA ATTTCTTTCT GAAATTAGTG AAAAAAACGC GCCTTTCAAA TTTTCATACT    2156
   AGTGATTGGT TTATTGCAGA TTATTTATTT CTAATTGTAC ATATTCTTGT TATTTTATAT    2216
   TATATGAAAG GGGTAAATCC AAATGCCACT CTACATACAG ATTCTGTAAC TGGCATAACG    2276
   ACCAGAGGTT TCACTTTTTC TTATGATTTT AACTACTTCG CCTCTTTTCA ATCCCAAGTA    2336
   TAAGGCTACA GGATCAGCTC TTTGAATTC                                      2365
(2) INFORMATION ZU SEQ ID NO: 12:
   (i) SEQUENZ CHARAKTERISTIKA:
      (A) LÄNGE: 244 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 12:
   Met Ser Leu Thr Pro Leu Cys Glu Asp Leu Pro Gln Phe Leu Gln Asn
     1               5                  10                  15
   Tyr Leu Pro Asn Ala Gly Gln Thr Glu Asn Thr Ile Val Pro Phe Val
                20                  25                  30
   Thr Leu Thr Tyr Ala Gln Ser Leu Asp Ala Arg Val Ser Arg Gly Pro
            35                  40                  45
   Gly Val Arg Thr Thr Ile Ser His Pro Glu Thr Lys Thr Met Thr His
        50                  55                  60
   Tyr Leu Arg His His His Asp Gly Ile Leu Val Gly Ser Gly Thr Val
    65                  70                  75                  80
   Leu Ala Asp Asn Pro Gly Leu Asn Cys Lys Trp Gly Pro Asp Pro Ala
                    85                  90                  95
   Ala Asn Ser Pro Arg Pro Ile Ile Ile Asp Thr Lys Gln Lys Trp Arg
               100                 105                 110
   Phe Asp Gly Ser Lys Met Gln Glu Leu Phe Ile Lys Arg Gln Gly Lys
           115                 120                 125
   Pro Pro Ile Val Val Val Thr Ser Glu Pro Ile Ile Lys Glu Gln His
       130                 135                 140
   Val Asp Tyr Ala Ile Cys Pro Ile Asn Asp Thr Thr Lys Leu Val Asp
   145                 150                 155                 160
   Trp Lys Lys Leu Phe Glu Ile Leu Lys Glu Glu Phe Asn Ile Arg Ser
                   165                 170                 175
   Val Met Val Glu Gly Gly Ala Asn Val Ile Asn Gln Leu Leu Leu Arg
               180                 185                 190
   Ser Asp Ile Val Asn Ser Leu Ile Ile Thr Ile Gly Ser Thr Phe Leu
           195                 200                 205
   Gly Ser Ser Gly Thr Glu Val Ser Pro Pro Gln Thr Val Asn Leu Lys
       210                 215                 220
   Asp Met Ser Trp Trp Lys Gly Ile Thr Asp Val Val Leu Cys Ala Arg
   225                 230                 235                 240
   Leu Ala Asp Asp

## Beschreibung

Die vorliegende Erfindung betrifft die Gene für Riboflavin-Biosynthese in Hefe, die damit codierten Proteine sowie gentechnische Verfahren zur Herstellung von Riboflavin unter Verwendung dieser Gene und Genprodukte.

Die Herstellung von Riboflavin durch Fermentation von Pilzen wie Eremothecium ashbyii oder Ashbya gossypii ist bekannt (The Merck Index, Windholz et al., eds. Merck & Co., Seite 1183, 1983).

In der EP 405370 sind Riboflavin-überproduzierende Bakterienstämme beschrieben, die durch Transformation der Riboflavin-Biosynthese-Gene aus Bacillus subtilis erhalten wurden.

Da die Genetik der Riboflavin-Biosynthese in Bakterien und Eukaryonten verschieden ist, sind die oben erwähnten Gene aus Bacillus subtilis nicht für ein rekombinantes Herstellverfahren für Riboflavin mit eukaryontischen Produktionsorganismen wie Saccharomyces cerevisiae oder Ashbya gossypii geeignet.

Von Revuelta et al. (From genes to bioproducts, BIOTEC 1990, Seite 117-122) werden die Isolierung der Gene für rib 3 und rib 5 aus Hefe beschrieben.
Es bestand daher die Aufgabe, die Riboflavin-Biosynthese Gene aus einem Eukaryonten zu isolieren, um damit ein rekombinantes Herstellverfahren für Riboflavin in einem eukaryontischen Produktionsorganismus bereitzustellen.

Demgemäß wurden in der Hefe Saccharomyces cerevisiae sechs Gene (rib-Gene), die für Enzyme der Riboflavin-Biosynthese ausgehend von GTP codieren, gefunden und isoliert.

Die Gene und ihre Genprodukte (Polypeptide) sind im Sequenzprotokoll mit ihrer Primärstruktur aufgeführt und haben folgende Zuordnung:
SEQ ID NO 1 : rib 1-Gen
SEQ ID NO 2 : rib 1-Genprodukt (GTP-cyclohydrolase II)
SEQ ID NO 3 : rib 2-Gen
SEQ ID NO 4 : rib 2-Genprodukt (DRAP-Deaminase)
SEQ ID NO 7 : rib 4-Gen
SEQ ID NO 8 : rib 4-Genprodukt (DMRL-Synthase)
SEQ ID NO 11: rib 7-Gen
SEQ ID NO 12: rib 7-Genprodukt (HTP-Reductase)

Guanosintriphosphat (GTP) wird durch GTP-Cyclohydrolase II (rib 1-Genprodukt) zu 2,5-Diamino-6-ribosylamino-4-(3H)-pyrimidin-5-phosphat umgewandelt. Diese Verbindung wird anschließend durch rib 7-Genprodukt zu 2,5-Diamino-ribitylamino-2,4 (1H,3H)-pyrimidin-5-phosphat reduziert und dann durch rib 2-Genprodukt zum 5-Amino-6-ribitylamino-2,4 (1H,3H)-pyrimidindion deaminiert. Anschließend wird in einer rib 4-Genprodukt katalysierten Reaktion die C4-Verbindung DBP hinzugefügt und es entsteht 6,7-Dimethyl-8-ribityllumazin (DMRL), aus dem in der rib 5-Genprodukt katalysierten Reaktion Riboflavin entsteht. Die C4-Verbindung DBP (L-3,4-Dihydroxy-2-butanon-4-phosphat) wird aus D-Ribulose-5-phosphat in einer rib 3-Genprodukt katalysierten Reaktion gebildet.

Die in SEQ ID NO 1,3,7 ,11 beschriebenen DNA-Sequenzen codieren für die Polypeptide, die in SEQ ID NO 2,4,8,12 beschrieben sind. Außer den oben genannten DNA-Sequenzen sind auch solche geeignet, die infolge der Degeneration des genetischen Codes eine andere DNA Sequenz besitzen, jedoch für das gleiche Polypeptid codieren. Weiterhin sind auch solche DNA Sequenzen Gegenstand der Erfindung, die zu den oben genannten DNA Sequenzen zu 90 oder mehr Prozent homolog sind und für Genprodukte mit gleicher biologischer Aktivität codieren. Solche DNA-Sequenzen lassen sich ausgehend von den in SEQ ID No 1, 3, 7, 9, 11 beschriebenen DNA-Sequenzen, beispielsweise mit üblichen Hybridisierverfahren oder der PCR-Technik aus anderen Eukaryonten als Saccharomyces cerevisiae isolieren.

Weiterhin sind Gegenstand der Erfindung Expressionsvektoren, die eine oder mehrere der erfindungsgemäßen DNA-Sequenzen enthalten.

Ebenso gehören die mit den erfindungsgemäßen DNA-Sequenzen oder Expressionsvektoren transformierten Wirtsorganismen zum Gegenstand der Erfindung. Bevorzugt werden als Wirtsorganismen eukaryontische Organismen, besonders bevorzugt solche der Gattung Saccharomyces oder Ashbya verwendet.

Weiterhin gehört zur Erfindung ein rekombinantes Herstellverfahren für Riboflavin, in dem die erfindungsgemäßen transformierten Wirtsorganismen in an sich bekannter Weise durch Fermentation gezüchtet werden und das während der Fermentation gebildete Riboflavin aus dem Fermentationsmedium isoliert und gegebenenfalls gereinigt wird.

Die rib-Gene und -Genprodukte lassen sich wie im Beispiel und im Sequenzprotokoll beschrieben isolieren und charakterisieren.

### Beispiel

### Klonierung der Riboflavin-Biosynthese Gene (rib-Gene) aus Hefe

Der Hefestamm JC2a, der mehrere geeignete Selektionsmarker für die Transformation enthält, (MatO, his3A1, leu2-3, 112, ura3-52) wurde durch Behandlung mit EMS mutagenisiert mit dem Ziel, rib-Mutationen einzuführen. Akkumulation, Komplementation und Wachstumstests von Riboflavin-Auxotrophen, die von Replica-Platten isoliert worden waren, bestätigten, daß die Isolate mit der Bezeichnung AJ 126, AJ 122, JA 118, AJ21, AJ18 und AJ12 jeweils in einem rib-Gen (rib1, rib2, rib3, rib4, rib5 und rib7) betroffen waren.

Um die entsprechenden Wildtyp Kopien der sechs rib-Gene zu erhalten wurde jede der obengenannten rib-Mutanten mit 50-100 µg DNA aus einer genomischen Hefe-Bibliothek transformiert.

Die Hefe-Bibliothek war in dem Centromer-Vektor YCp50 angelegt worden und kann in dieser Form von ATCC (Nr. 37415) erhalten werden. Die Transformation wurde nach der Lithiumacetat Methode (Ito et al., J. Bacteriol. 153,163, 1983) durchgeführt. Die Transformanden wurden auf gleichzeitige Uracil- und Riboflavin Prototrophie auf einem synthetischen Vollmedium ohne Uracil und Riboflavin selektioniert. Die Frequenz betrug je nach Empfängerstamm 10⁻⁴ bis 10⁻⁵. Die Plasmide wurden von den positiven Transformanden isoliert, indem E. coli mit der gesamten Hefe DNA transformiert wurde und auf Ampicillinresistenz selektioniert wurde.

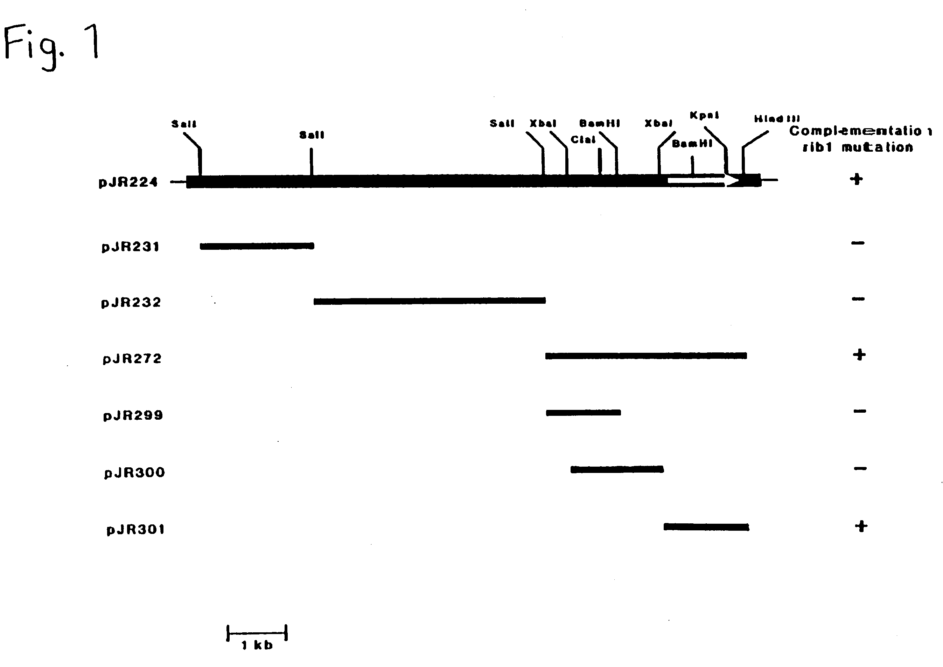

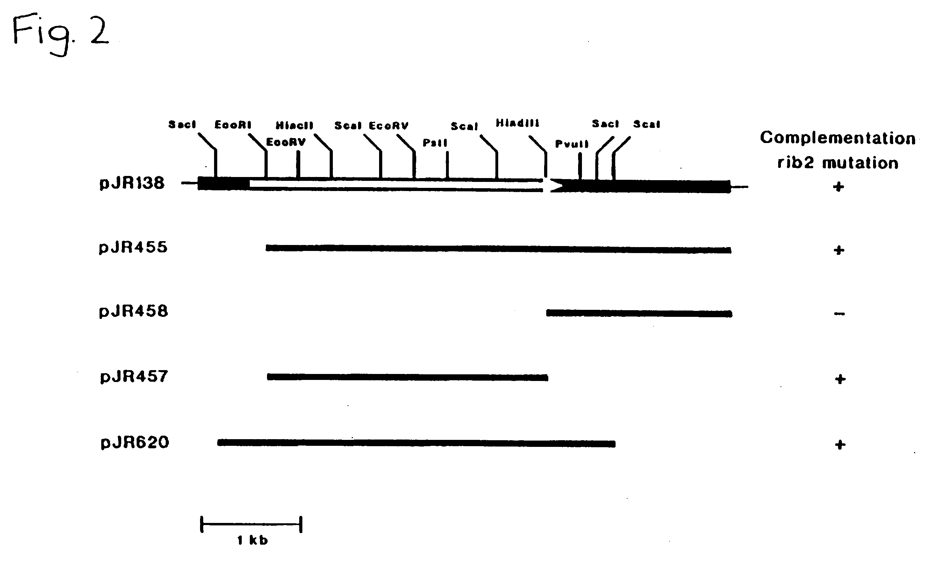

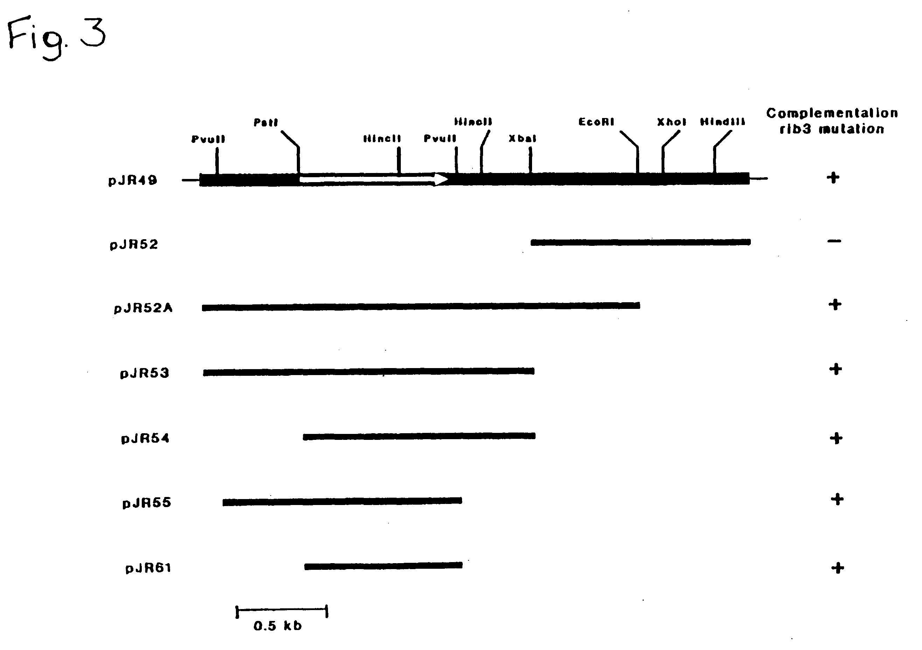

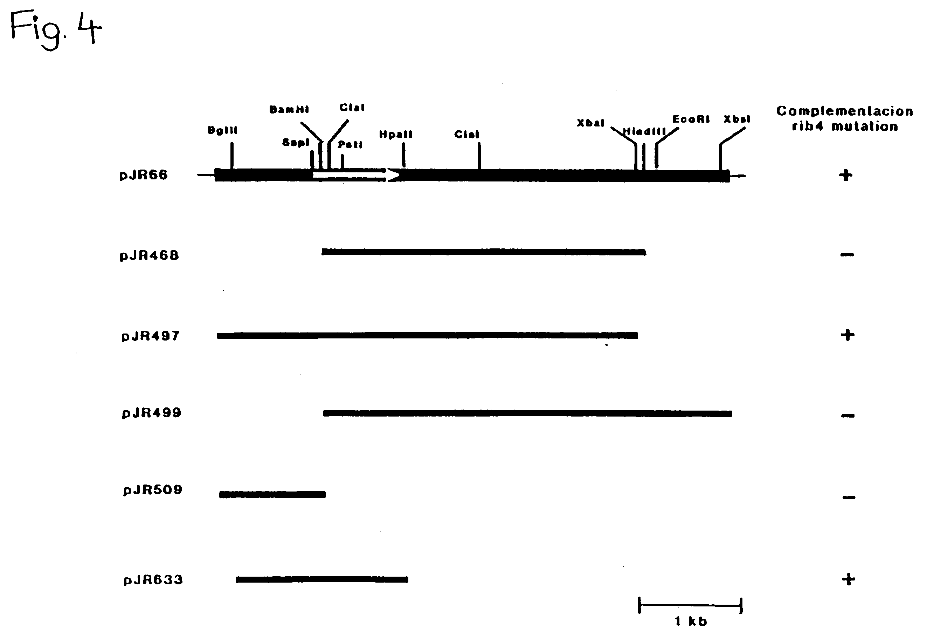

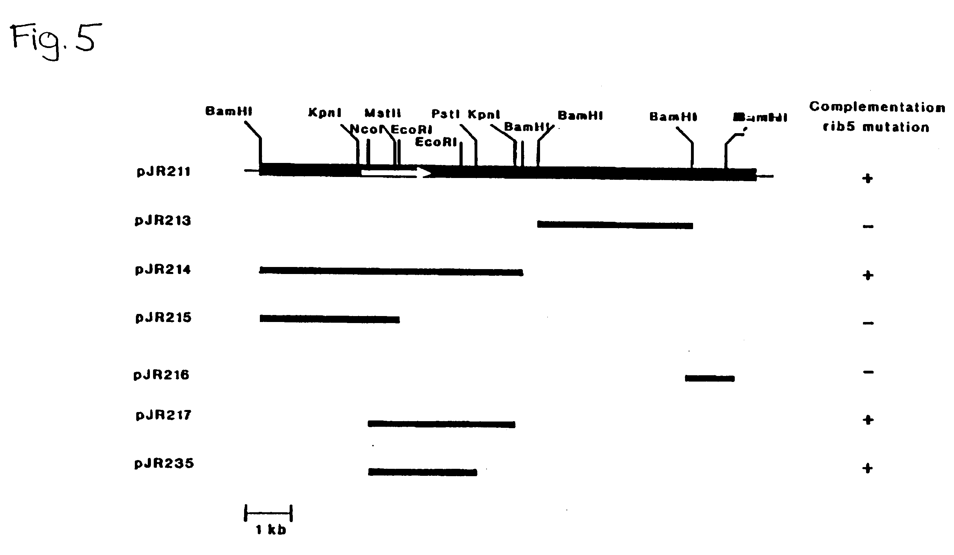

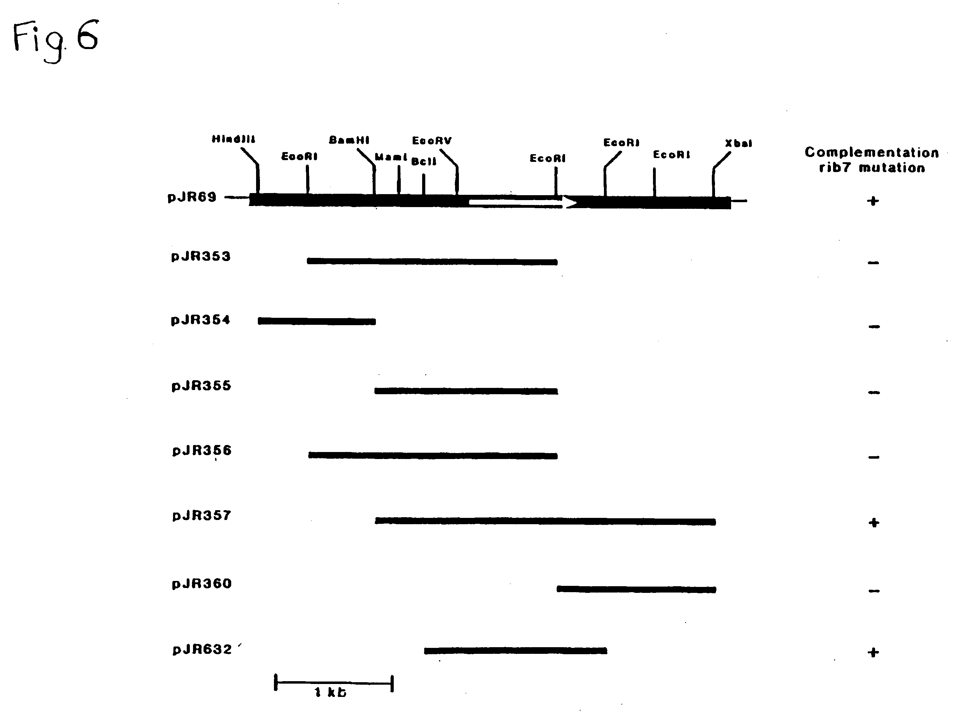

Die rib-komplementierenden Genbereiche wurden durch Subklonierung lokalisiert. Wie aus Fig. 1 ersichtlich ist, wurde das rib1 Gen auf einem 1,7 kb XbaI - HindIII Fragment lokalisiert, das in dem multi-copy Vector YEp352 subkloniert wurde, und das Plasmid pJR301 ergab. Entsprechend wurde das rib2-Gen auf einem 2,7 kb SacI - SacI Fragment lokalisiert (subkloniert in pJR 620, Fig. 2), das rib4-Gen auf einem 1,7 kb Bgl II - HpaII Fragment (subkloniert in pJR633, Fig.4) und das rib7-Gen auf einem 1,6 kb BclI - EcoRI Fragment (subkloniert in pJR632, Fig.6).

Die Insertionen der entsprechenden pJR-Plasmide wurden mit Hilfe der Dideoxymethode von Sanger auf beiden Strängen sequenziert. Die Analyse von codierenden Bereichen ergab in allen Fällen offene Leserahmen von mehr als 500 bp.

| Gen | DNA (bp) | Polypeptid (AS) |
|---|---|---|
| rib 1 | SEQ ID NO 1 (1747) | SEQ ID NO 2 (345) |
| rib 2 | SEQ ID NO 3 (3086) | SEQ ID NO 4 (591) |
| rib 4 | SEQ ID NO 7 (1300) | SEQ ID NO 8 (169) |
| rib 7 | SEQ ID NO 11 (2365) | SEQ ID NO 12 (244) |

## Patentansprüche

1. DNA-Sequenz, die für ein Polypeptid mit der in SEQ ID NO 2 dargestellten Aminosäuresequenz codiert.

2. DNA-Sequenz, die für ein Polypeptid mit der in SEQ ID NO 4 dargestellten Aminosäuresequenz codiert.

3. DNA-Sequenz, die für ein Polypeptid mit der in SEQ ID NO 8 dargestellten Aminosäuresequenz codiert.

4. DNA-Sequenz, die für ein Polypeptid mit der in SEQ ID NO 12 dargestellten Aminosäuresequenz codiert.

5. Expressionsvektor, enthaltend eine oder mehrere DNA-Sequenzen gemäß Anspruch 1 bis 4.

6. Rekombinantes Herstellverfahren für Riboflavin, dadurch gekennzeichnet, daß ein Expressionsvektor gemäß Anspruch 5 verwendet wird.

7. Wirtsorganismus der mit einem Expressionsvektor gemäß Anspruch 5 transformiert worden ist.

## Claims

1. A DNA sequence which codes for a polypeptide having the amino acid sequence shown in SEQ ID NO 2.

2. A DNA sequence which codes for a polypeptide having the amino acid sequence shown in SEQ ID NO 4.

3. A DNA sequence which codes for a polypeptide having the amino acid sequence shown in SEQ ID NO 8.

4. A DNA sequence which codes for a polypeptide having the amino acid sequence shown in SEQ ID NO 12.

5. An expression vector containing one or more DNA sequences as claimed in claims 1 to 4.

6. A recombinant preparation process for riboflavin, which comprises using an expression vector as claimed in claim 5.

7. A host organism which has been transformed using an expression vector as claimed in claim 5.

## Revendications

1. Séquence d'ADN qui code pour un polypeptide qui possède la séquence d'aminoacides présentée dans SEQ ID NO 2.

2. Séquence d'ADN qui code pour un polypeptide qui possède la séquence d'aminoacides présentée dans SEQ ID NO 4.

3. Séquence d'ADN qui code pour un polypeptide qui possède la séquence d'aminoacides présentée dans SEQ ID NO 8.

4. Séquence d'ADN qui code pour un polypeptide qui possède la séquence d'aminoacides présentée dans SEQ ID NO 12.

5. Vecteur d'expression, contenant une ou plusieurs séquences d'ADN selon les revendications 1 à 4

6. Procédé de préparation à recombinaison pour la riboflavine, caractérisé en ce que l'on utilise un vecteur d'expression selon la revendication 5.

7. Organisme hôte qui a été transformé avec un vecteur d'expression selon la revendication 5.
